# EUROPEAN PATENT SPECIFICATION

(11) **EP 2 334 844 B1**
(45) Date of publication and mention of the grant of the patent: **08.07.2015**
(21) Application number: 09816524.4
(22) Date of filing: 22.09.2009
(51) Int. Cl.: C25D 3/56, A61L 2/238, C23C 18/48, C25D 3/58, C25D 3/60, A61L 2/232

(54) **METHOD FOR THE ELECTROLYTIC PLATING OF AN ARTICLE, AND AN ELECTROLYTIC SOLUTION**
VERFAHREN ZUR ELEKTROLYTISCHEN PLATTIERUNG EINES ARTIKELS SOWIE ELEKTROLYSELÖSUNG DAFÜR
PROCÉDÉ POUR LE PLACAGE ÉLECTROLYTIQUE D'UN ARTICLE, ET SOLUTION ÉLECTROLYTIQUE

(30) Priority: 26.09.2008 SE 0850022
(43) Date of publication of application: 22.06.2011
(73) Proprietor: ASSA AB, 631 05 Eskilstuna (SE)
(72) Inventor: LILLSJÖ, Jarmo, S-632 26 Eskilstuna (SE); BOVIN, Perla, S-644 33 Torshälla (SE)
(74) Representative: Kransell & Wennborg KB
(86) International application number: PCT/SE2009/051054
(87) International publication number: WO 2010/036189

(56) References cited:
- EP-A1- 1 826 295
- WO-A1-2006/039479
- WO-A2-2004/006228
- GB-A- 592 443
- US-A- 2 530 967
- US-A- 5 614 327
- US-A1- 2003 118 624
- US-A1- 2005 224 425
- US-A1- 2006 165 943
- US-A1- 2006 180 552

## Description

### Field of the invention

The present invention relates to a method for the electrolytic plating of articles, as defined in the pre-characterizing part of Claim 1.

### Background to the invention

Products are often subjected to a surface treatment in order to produce certain desirable characteristics in the finished product. For example, products can be treated to make their surfaces resistant to corrosion, chemicals and wear and tear, as well as to impart e.g. a certain quality or appearance to them.

With increased knowledge of the way infections spread and with a greater health consciousness, surfaces can nowadays be treated in order to confer antibacterial properties on them. These properties are in ever increasing demand and can be obtained for example by treating the surface of a product with a silver solution.

A difficulty with providing a surface with antibacterial properties is the introduction of such a processing stage into the surface treatment process. Another difficulty is to maintain the other desirable characteristics, in other words, to combine a processing stage that is to ensure antibacterial properties with a processing stage that provides a feature such as for example a glossy surface.

An example of surface treatment methods is electroplating, where electrolysis is used to coat a metal object with a layer of another metal. For electroplating products differing in appearance, the process must be adjusted precisely to suit the product in question. For example, a product with many holes may require a greater current intensity in the electrolysis than a product with a flat surface, and several factors may need to be considered to ensure that the metal covers the article uniformly, creating a film of the same thickness throughout in order to ensure a high surface homogeneity. The process is further complicated when the surface treatment includes another processing stage, designed to provide antibacterial properties.

### Aim and main features of the invention

One of the aims of the present invention is to provide a surface treatment method that solves the above problems. More specifically, one of the aims of the invention is to provide both a method of electrolytic plating and an electrolyte solution for conferring antibacterial properties to a surface.

Another aim of the present invention is to provide a surface treatment method in which the processing stage that is intended for conferring antibacterial properties on a surface does not interfere with the results of the surface treatment process as regards the other characteristics.

Yet another aim of the present invention is to provide a stable surface treatment method that gives uniform and predictable results for all products.

These and other aims are achieved according to the present invention by a plating method specified in the characterizing part of Claim 1.

The above aims are achieved according to the present invention by a method for the electrolytic plating of an article. This method includes an alloying stage, in which the articles are coated with an alloy comprising zinc, copper and tin by immersing them into an electrolyte solution that contains salts of zinc, copper and tin. The method is characterized in that the article is also provided with antibacterial properties in the same alloying stage, by introducing a silver compound into the electrolyte solution. The silver compound is a silver salt and the amount in which the silver salt is added lies within the range of 0.5-1.5 g per litre electrolyte solution. Owing to the presence of the silver compound in the electrolytic bath used in the alloying stage, the surface of the article also acquires antibacterial properties here, thanks to the silver that is incorporated in the plating layer. The method can be used to provide an article with a surface that is functional, has the required aesthetic characteristics and possesses antibacterial properties as well. Articles that are handled by a large number of people, such as for example door handles, door knobs, etc. are particularly suitable for treatment according to the present invention, since an antibacterial surface is a most valuable property for reducing the spread of infections. The other characteristics of the surface are not affected, and the articles receive the desired, predictable appearance.

In one of the embodiments of the invention, the electrolyte solution used in the alloying stage contains metals in the following quantities per litre: 20 g of Sn, 7.8 g of Cu, 1.9 g of Zn, 26 g of KCN and 8 g of KOH. Owing to these specific amounts of the substances added to the electrolyte solution, the surface of the article acquires the required characteristics, and the addition of the antibacterial substance does not exert a notable effect on these characteristics.

The invention also relates to an electrolyte solution that is suitable for the electrolytic plating of metal articles according to the specified method, so the advantages mentioned above are obtained.

Further advantages are obtained from the various aspects of the invention, which are described in detail below.

### Detailed description of the embodiments of the invention

The word "article" is used in the following description to cover products that are obtained by the method specified here, especially products such as door handles, door knobs, locks etc., which are touched by many people, so an antibacterial surface for them is of great value in the reduction of the spread of infections. However, it should be mentioned here that the present invention can also be used for treating the surface of other types of products with a metal.

It is clear from the introductory description that numerous requirements must be satisfied in order to create the required surface on an article.

The Applicant is currently using a surface treatment method that can be regarded as an alternative to bright chromium plating, which gives both a silvery sheen and a high resistance to wear and tear. This surface treatment comprises a number of preparatory stages, an alloying stage and a number of finishing stages. The present invention is mainly related to the alloying stage.

This known electroplating process, used for conferring a metallic surface on various articles, therefore comprises some preparatory stages, such as degreasing, in which a large part of the oil is removed from the surface of the article by first forming an emulsion and then removing it. In such preparatory stages, the article in question is passed through for example a processing bath for biological degreasing, a processing bath for cathodic electrical degreasing and/or a processing bath for anodic electrical degreasing.

These are followed by the use of an activating bath, where the atoms on the metal surface to be coated by electroplating are activated, i.e. the surface of the article is made receptive to new metals. This is followed by an alkaline copper bath, in which the article is coated with a layer of copper. The latter is applied in order to create a layer that ensures a good adhesion and forms a barrier on the base material. The alkaline copper bath is followed by the use of another activating bath - this time one containing sulphuric acid (H₂SO₄)- in order to activate the copper layer. The article in question is then immersed into a copper bath once more, which contains acidic copper. This provides the article with a bright surface. The subsequent alloying stage, which will be described below, calls for a bright layer under it in order to ensure the right surface finish for the article.

The electroplating method that is considered to give the required surface is a "white bronze" process, i.e. an alloy formation between zinc (Zn), tin (Sn) and copper (Cu), called the "alloying stage" below. Salts of these metals, such as zinc cyanide, copper cyanide and tin stannate (potassium stannate), can also be used.

In the alloying stage, a Zn-Sn-Cu alloy is deposited on the surface of the article by electrolysis, giving a metallic surface that can be either bright or matt, resembling bright or matt chromium. The electrolyte solution used in this alloying stage contains ions of the metals Zn, Sn and Cu, which are to coat the surface of the article, and which are then conventionally electroplated onto the surface of the article. More specifically, an electrolytic precipitation of the metals Sn, Cu and Zn is carried out, and the metal ions present in the electrolytic bath are reduced on the surface of the cathode, i.e. on the article in question, forming the required metal surface.

This is followed by the finishing stages, which start with another activating bath, containing sodium fluoride, needed for the re-activation of the metal surface of the article. The article is then inserted into a processing bath containing trivalent chromium (Cr³⁺), a bath that preferably has a pH of 2.7 and is kept at a temperature of about 30-35°C. This stage makes the article resistant to corrosion. It can also be considered to seal the required layer thus obtained and to prevent the tin (Sn) from oxidation in the alloy deposited in the alloying stage. This oxidation would otherwise make the surface of the article turn dark during use, which must be prevented. The product is finally rinsed in warm water and dried in warm air at a temperature of about 60°C.

The article is also rinsed once or more often between any of the processing stages mentioned above. Thus, it is rinsed between the stages called preparatory stages above, then both before and after the alloying stage, and finally between the stages called finishing stages here.

The plating process described above does not include any nickel, which is an advantage in view of the ever increasing occurrence of nickel allergy.

However, it has been found that the plating process described above, and more specifically its alloying stage, lead to an article with a variable surface quality. The surface obtained has a certain degree of mattness instead of the required bright metallic appearance. According to the supplier, the various components of the electrolyte solution used in the alloying stage should be introduced in the following amounts:

| | |
|---|---|
| Sn (tin) | 20 g/l |
| Cu (copper) | 8 g/l |
| Zn (zinc) | 2 g/l |
| KCN (potassium cyanide) | 25 g/l |
| KOH (potassium hydroxide) | 10-15 g/l |
| Brightener | 1-3 ml/l |
| Wetting agent | 1-2 ml/l. |

Additives are conventionally incorporated in the bath, so that the process ensures the required characteristics for the products. More specifically, brighteners are added to provide the required gloss or sheen, and wetting agents are added to control the surface tension of the electrolyte solution.

With years of experience and after testing for a long time, the present Applicant has succeeded in improving the existing process. A better result regarding the surface layer of the articles is obtained by using different quantities for the constituents of the electrolyte solution used in the alloying stage. More uniform results have been obtained with the following composition:

| | |
|---|---|
| Sn | 21 g/l |
| Cu | 8 g/l |
| Zn | 2 g/l |
| KCN | 30 g/l |
| KOH | 7 g/l |
| Brightener | 6 ml/l |
| Wetting agent | 2 ml/l. |

The aim to obtain articles with an antibacterial surface called for more work, involving the testing of suitable compositions. An antibacterial surface is obtained according to the present invention by adding silver to the bath used in the alloying stage. This silver additive comprises silver compounds, such as silver salts. A commercially available product, "GC 100", made and marketed by Polygiene^{®}, has been found suitable for use as this silver additive. This GC 100 product contains a composition of silver, magnesium, aluminium and phosphorus.

The use of a silver additive in the surface treatment method introduces a further consideration. One aspect of it is that the silver additive increases the cost, so its amount should be minimized to ensure a cost-effective process. On the other hand, the silver additive must be used in a sufficient amount to ensure the required antibacterial effect.

It is also important that the silver additive used should not interfere with, or have a negative effect on, the surface treatment process, otherwise it is impossible to obtain the required surface finish for the articles. It has been found that a suitable amount of silver additive lies within the interval of 0.5-1.5 g per litre electrolyte solution.

Tests and experiments have led to the following adjustments to the modified electrolyte solution described above for use in the said alloying stage, this composition giving a much better result:

| | |
|---|---|
| Sn | 20 g/l |
| Cu | 7.8 g/l |
| Zn | 1.9 g/l |
| KCN | 26 g/l |
| KOH | 8 g/l |
| Brightener | 6 ml/l |
| Wetting agent | 2 ml/l |
| Ag additive | 1 g/l. |

As mentioned before, the electroplating process may have to be modified to suit the product in question if it is to give products that differ in their appearance. One or more of the values may be different from the above optimum amounts without negatively affecting the properties of the article. The above amounts can therefore be modified within the following ranges:

| | |
|---|---|
| Sn | 19-21 g/l |
| Cu | 7-9 g/l |
| Zn | 1-3 g/l |
| KCN | 25-27 g/l |
| KOH | 7-9 g/l. |

In the alloying stage, the article that is to be plated is immersed in a solution containing the salts of the constituents mentioned above, which are dissolved in distilled water in the usual way. The article is immersed in this electrolyte solution after carrying out the various preparatory stages, the result being a bright metallic surface reminiscent of bright chromium. The surface can of course be brushed to make it matt in order to obtain a matt metallic surface similar to matt chromium.

The incorporation of the silver additive (Ag additive) in the electrolyte solution used in the alloying stage gives the article a surface that also possesses antibacterial properties, owing to the silver deposited in the layer that has been plated on it.

The other required properties of the surface of the article are also obtained, thanks to the amounts in which the various substances are introduced into the electrolytes.

## Claims

1. Method for the electrolytic plating of an article, comprising an alloying stage, in which the said article is given a coating of an alloy containing zinc (Zn), copper (Cu) and tin (Sn), by immersing it in an electrolyte solution containing salts of zinc, copper and tin, **characterized in that** the said article is also provided with antibacterial properties in the same alloying stage by introducing a silver compound into the said electrolyte solution, where the said silver compound is a silver salt, and the amount in which the silver salt is added lies within the range of 0.5-1.5 g per litre of electrolyte solution.

2. Method according to Article 1, where the said silver compound is GC 100, made by Polygiene^{®}.

3. Method for electrolytic plating according to Claim 1 or 2, where one or more of the following preparatory stages is/are included before the said alloying stage: biological degreasing, cathodic electrical degreasing, anodic electrical degreasing, an activating stage in which the atoms on the surface of the article are activated, and rinsing.

4. Method for electrolytic plating according to any one of the preceding claims, where one or more of the following stages is/are also included after the said alloying stage: an activating stage in which the atoms on the surface of the article are activated, immersion in a processing bath containing trivalent chromium (Cr), rinsing and drying.

5. Method for electrolytic plating according to any one of the preceding claims, where the said electrolyte solution also contains potassium cyanide (KCN) and potassium hydroxide (KOH).

6. Method for electrolytic plating according to Claim 5, where the said electrolyte solution contains the said constituents in the following amounts:
| | |
|---|---|
| Sn | 19-21 g/l |
| Cu | 7-9 g/l |
| Zn | 1-3 g/l |
| KCN | 25-27 g/l |
| KOH | 7-9 g/l. |

7. Method for electrolytic plating according to Claim 5 or 6, where the said electrolyte solution contains the said constituents in the following amounts:
| | |
|---|---|
| Sn | 20 g/l |
| Cu | 7.8 g/l |
| Zn | 1.9 g/l |
| KCN | 26 g/l |
| KOH | 8 g/l. |

8. Method for electrolytic plating according to any one of the preceding claims, where the said electrolyte solution also contains brighteners and/or wetting agents.

9. Method for electrolytic plating according to Claim 8, where the brightener is added in an amount of 6 ml/l, and the wetting agent is added in an amount of 2 ml/l.

10. Electrolyte solution for plating an article, which electrolyte solution contains salts of zinc, copper and tin, **characterized in that** the said electrolyte solution also contains a silver compound comprising a silver salt, the amount of the said silver salt lying in the range of 0.5-1.5 g per litre of electrolyte solution.

11. Electrolyte solution according to Claim 10, where the said silver compound is GC 100, made by Polygiene^{®}.

12. Electrolyte solution according to Claim 10 or 11, comprising 1 g of silver salt per litre.

13. Electrolyte solution according to any one of Claims 10-12, also containing potassium cyanide (KCN) and potassium hydroxide (KOH).

14. Electrolyte solution according to Claim 13, containing the said constituents in the following amounts:
| | |
|---|---|
| Sn | 19-21 g/l |
| Cu | 7-9 g/l |
| Zn | 1-3 g/l |
| KCN | 25-27 g/l |
| KOH | 7-9 g/l. |

15. Electrolyte solution according to Claim 13 or 14, containing the said constituents in the following amounts:
| | |
|---|---|
| Sn | 20 g/l |
| Cu | 7.8 g/l |
| Zn | 1.9 g/l |
| KCN | 26 g/l |
| KOH | 8 g/l. |

16. Electrolyte solution according to any one of Claims 10-15, also containing brighteners and wetting agents.

17. Electrolyte solution according to Claim 16, where the brightener is added in an amount of 6 ml/l, and the wetting agent is added in an amount of 2 ml/l.

## Patentansprüche

1. Verfahren zur elektrolytischen Plattierung eines Artikels , umfassend einen Legierungsschritt, bei dem der Artikel eine Beschichtung aus einer Legierung enthaltend Zink (Zn), Kupfer (Cu) und Zinn (Sn) erhält, dadurch, dass er in eine Elektrolytlösung enthaltend Salze von Zink, Kupfer und Zinn eingetaucht wird, **dadurch gekennzeichnet, dass** der Artikel in demselben Legierungsschritt ebenfalls mit antibakteriellen Eigenschaften ausgestattet wird durch das Einbringen einer Silberverbindung in die Elektrolytlösung, wobei die Silberverbindung ein Silbersalz ist, und die Menge, in der das Silbersalz zugegeben wird im Bereich von 0,5-1,5 g pro Liter der Elektrolytlösung liegt.

2. Verfahren gemäß Artikel 1, wobei die Silberverbindung GC 100, hergestellt von Polygiene^{®}, ist.

3. Verfahren zur elektrolytischen Plattierung gemäß Anspruch 1 oder 2, wobei einer oder mehrere der folgenden, vorbereitenden Schritte vor dem Legierungsschritt eingeschlossen ist/sind: biologisches Entfetten, kathodisches elektrisches Entfetten, anodisches elektrisches Entfetten, ein Aktivierungsschritt, bei dem die Atome auf der Oberfläche des Artikels aktiviert werden, und Spülen.

4. Verfahren zur elektrolytischen Plattierung gemäß einem der vorangegangenen Ansprüche, wobei einer oder mehrere der folgenden Schritte nach dem Legierungsschritt eingeschlossen ist/sind: ein Aktivierungsschritt, bei dem die Atome auf der Oberfläche des Artikels aktiviert werden, Eintauchen in ein Prozessbad enthaltend dreiwertiges Chrom (Cr), Spülen und Trocknen.

5. Verfahren zur elektrolytischen Plattierung gemäß einem der vorangegangenen Ansprüche, wobei die Elektrolytlösung ebenfalls Kaliumcyanid (KCN) und Kaliumhydroxid (KOH) enthält.

6. Verfahren zur elektrolytischen Plattierung gemäß Anspruch 5, wobei die Elektrolytlösung diese Bestandteile in den folgenden Mengen enthält:
| | |
|---|---|
| Sn | 19-21 g/l |
| Cu | 7-9 g/l |
| Zn | 1-3 g/l |
| KCN | 25-27 g/l |
| KOH | 7-9 g/l. |

7. Verfahren zur elektrolytischen Plattierung gemäß Anspruch 5 oder 6, wobei die Elektrolytlösung diese Bestandteile in den folgenden Mengen enthält:
| | |
|---|---|
| Sn | 20 g/l |
| Cu | 7,8 g/l |
| Zn | 1,9 g/l |
| KCN | 26 g/l |
| KOH | 8 g/l. |

8. Verfahren zur elektrolytischen Plattierung gemäß einem der vorangegangenen Ansprüche, wobei die Elektrolytlösung ebenfalls Aufheller und/oder Benetzungsmittel enthält.

9. Verfahren zur elektrolytischen Plattierung gemäß Anspruch 8, wobei der Aufheller in einer Menge von 6 ml/l zugegeben wird und das Benetzungsmittel in einer Menge von 2 ml/l zugegeben wird.

10. Elektrolytlösung zur Plattierung eines Artikels, wobei die Elektrolytlösung Salze von Zink, Kupfer und Zinn enthält, **dadurch gekennzeichnet, dass** die Elektrolytlösung ebenfalls eine Silberverbindung umfassend ein Silbersalz enthält, wobei die Menge des Silbersalzes im Bereich von 0,5-1,5 g pro Liter der Elektrolytlösung liegt.

11. Elektrolytlösung gemäß Anspruch 10, wobei die Silberverbindung GC 100, hergestellt von Polygiene^{®}, ist.

12. Elektrolytlösung gemäß Anspruch 10 oder 11 umfassend 1 g Silbersalz pro Liter.

13. Elektrolytlösung gemäß irgendeinem der Ansprüche 10-12, ebenfalls enthaltend Kaliumcyanid (KCN) und Kaliumhydroxid (KOH).

14. Elektrolytlösung gemäß Anspruch 13, enthaltend diese Bestandteile in den folgenden Mengen:
| | |
|---|---|
| Sn | 19-21 g/l |
| Cu | 7-9 g/l |
| Zn | 1-3 g/l |
| KCN | 25-27 g/l |
| KOH | 7-9 g/l. |

15. Elektrolytlösung gemäß Anspruch 13 oder 14, enthaltend diese Bestandteile in den folgenden Mengen:
| | |
|---|---|
| Sn | 20 g/l |
| Cu | 7,8 g/l |
| Zn | 1,9 g/l |
| KCN | 26 g/l |
| KOH | 8 g/l. |

16. Elektrolytlösung gemäß irgendeinem der Ansprüche 10-15, ebenfalls enthaltend Aufheller und Benetzungsmittel.

17. Elektrolytlösung gemäß Anspruch 16, wobei der Aufheller in einer Menge von 6 ml/l zugegeben wird und das Benetzungsmittel in einer Menge von 2 ml/l zugegeben wird.

## Revendications

1. Procédé de dépôt électrolytique d'un article, comprenant une étape d'alliage, au cours de laquelle ledit article reçoit un revêtement d'un alliage contenant du zinc (Zn), du cuivre (Cu) et de l'étain (Sn), par immersion dans une solution électrolytique contenant des sels de zinc, de cuivre et d'étain, **caractérisé en ce que** ledit article se voit aussi conférer des propriétés antibactériennes au cours de la même étape d'alliage par introduction d'un composé de l'argent dans ladite solution électrolytique, ledit composé de l'argent étant un sel d'argent, et la quantité ajoutée du sel d'argent étant comprise dans la plage de 0,5 à 1,5 g par litre de solution électrolytique.

2. Procédé selon la revendication 1, dans lequel ledit composé de l'argent est le GC 100, fabriqué par Polygiene®.

3. Procédé de dépôt électrolytique selon la revendication 1 ou 2, dans lequel une ou plusieurs des étapes préparatoires suivantes sont incluses avant ladite étape d'alliage : dégraissage biologique, dégraissage électrique cathodique, dégraissage électrique anodique, une étape d'activation au cours de laquelle les atomes se trouvant sur la surface de l'article sont activés, et rinçage.

4. Procédé de dépôt électrolytique selon l'une quelconque des revendications précédentes, dans lequel une ou plusieurs des étapes suivantes sont incluses après ladite étape d'alliage : une étape d'activation au cours de laquelle les atomes se trouvant sur la surface de l'article sont activés, immersion dans un bain de traitement contenant du chrome trivalent (Cr), rinçage et séchage.

5. Procédé de dépôt électrolytique selon l'une quelconque des revendications précédentes, dans lequel ladite solution électrolytique contient aussi du cyanure de potassium (KCN) et de l'hydroxyde de potassium (KOH).

6. Procédé de dépôt électrolytique selon la revendication 5, dans lequel ladite solution électrolytique contient lesdits constituants en les quantités suivantes :
| | |
|---|---|
| Sn | 19-21 g/l |
| Cu | 7-9 g/l |
| Zn | 1-3 g/l |
| KCN | 25-27 g/l |
| KOH | 7-9 g/l. |

7. Procédé de dépôt électrolytique selon la revendication 5 ou 6, dans lequel ladite solution électrolytique contient lesdits constituants en les quantités suivantes :
| | |
|---|---|
| Sn | 20 g/l |
| Cu | 7,8 g/l |
| Zn | 1,9 g/l |
| KCN | 26 g/l |
| KOH | 8 g/l. |

8. Procédé de dépôt électrolytique selon l'une quelconque des revendications précédentes, dans lequel ladite solution électrolytique contient aussi des azurants et/ou des agents mouillants.

9. Procédé de dépôt électrolytique selon la revendication 8, dans lequel l'azurant est ajouté en une quantité de 6 ml/l, et l'agent mouillant est ajouté en une quantité de 2 ml/l.

10. Solution électrolytique pour placage d'un article, laquelle solution électrolytique contenant des sels de zinc, de cuivre et d'étain, **caractérisée en ce que** ladite solution électrolytique contient aussi un composé de l'argent comprenant un sel d'argent, la quantité dudit sel d'argent étant comprise dans la plage de 0,5 à 1,5 g par litre de solution électrolytique.

11. Solution électrolytique selon la revendication 10, dans laquelle ledit composé de l'argent est le GC 100, fabriqué par Polygiene®.

12. Solution électrolytique selon la revendication 10 ou 11, comprenant 1 g de sel d'argent par litre.

13. Solution électrolytique selon l'une quelconque des revendications 10 à 12, contenant aussi du cyanure de potassium (KCN) et de l'hydroxyde de potassium (KOH).

14. Solution électrolytique selon la revendication 13, contenant lesdits constituants en les quantités suivantes :
| | |
|---|---|
| Sn | 19-21 g/l |
| Cu | 7-9 g/l |
| Zn | 1-3 g/l |
| KCN | 25-27 g/l |
| KOH | 7-9 g/l. |

15. Solution électrolytique selon la revendication 13 ou 14, contenant lesdits constituants en les quantités suivantes :
| | |
|---|---|
| Sn | 20 g/l |
| Cu | 7,8 g/l |
| Zn | 1,9 g/l |
| KCN | 26 g/l |
| KOH | 8 g/l. |

16. Solution électrolytique selon l'une quelconque des revendications 10 à 15, contenant aussi des azurants et des agents mouillants.

17. Solution électrolytique selon la revendication 16, dans laquelle l'azurant est ajouté en une quantité de 6 ml/l, et l'agent mouillant est ajouté en une quantité de 2 ml/l.
